# EUROPEAN PATENT SPECIFICATION

(11) **EP 1 715 893 B1**
(45) Date of publication and mention of the grant of the patent: **15.07.2009**
(21) Application number: 05700976.3
(22) Date of filing: 17.01.2005
(51) Int. Cl.: A61K 47/00

(54) **DIRECT COMPRESSION FORMULATION AND PROCESS**
DIREKTKOMPRIMIERTE FORMULIERUNG UND VERFAHREN
FORMULATION A COMPRESSION DIRECTE ET PROCEDE CORRESPONDANT

(30) Priority: 20.01.2004 US 537706 P; 25.08.2004 US 604274 P
(43) Date of publication of application: 02.11.2006
(73) Proprietor: Novartis Pharma AG, 4002 Basel (CH); NOVARTIS-PHARMA GMBH, 1230 Vienna (AT)
(72) Inventor: KOWALSKI, James, Belle Mead, NJ 08502 (US); PARTHIBAN, Lakshman, Jayanth, Cedar Knolls, NJ 07927 (US); PATEL, Arun, P., Succasunna, NJ 07876 (US)
(74) Representative: Hillebrand, Dirk
(86) International application number: PCT/EP2005/000400
(87) International publication number: WO 2005/067976

(56) References cited:
- EP-A- 1 537 880
- WO-A-03/057195
- JP-A- 2003 327 532
- US-A- 4 452 579
- US-A1- 2003 078 247
- US-A1- 2003 087 950
- US-B1- 6 548 481

## Description

This invention relates to tablets especially tablets formed by direct compression of a dipeptidylpeptidase IV (DPP-IV) inhibitor compound, a process for the preparation thereof, to new pharmaceutical formulations as claimed in claim 1 . The invention relates further to a process for preparing the tablets by blending the active ingredient and specific excipients into the new formulations and then directly compressing the formulations into the direct compression tablets.

DPP-IV inhibitors are known in the art. For example, DPP-IV inhibitors are in each case generically and specifically disclosed e.g. in WO 98/19998,DE19616 486 A1, WO 00/34241, WO 95/15309, WO 01/72290, WO 01/52825, WO 9310127, WO 9925719, WO 9938501, WO 9946272, WO 9967278 and WO 9967279.

WO 9819998 discloses N-(N'-substituted glycyl)-2-cyano pyrrolidines, in particular 1-[2-[5-Cyanopyridin-2-yl] amino]- ethylamino] acetyl-2-cyano- (S)- pyrrolidine. Preferred compounds described in WO03/002553 are listed on pages 9 to 11. Published patent application WO 0034241 and published patent US 6110949 disclose N-substituted adamantyl-amino-acetyl-2-cyano pyrrolidines and N-(substituted glycyl)-4-cyano pyrrolidines respectively. DPP-IV inhibitors of interest are specially those cited in claims 1 to 4. In particular these applications describe the compound 1-[[(3-Hydroxy-1-adamantyl) amino]acetyl]-2-cyano-(S)-pyrrolidine (also known as LAF237).

LAF237 is the compound of the invention and is specifically disclosed in Example 1 of WO 00/34241. The preferred formulations for the administration of LAF237 are described in the US provisional application No. 60/604274.

The DPP-IV inhibitor compound, and its corresponding pharmaceutically acceptable acid addition salts, may be combined with one or more pharmaceutically acceptable carriers and, optionally, one or more other conventional pharmaceutical adjuvants and administered enterally, e.g., orally, in the form of tablets, capsules, caplets, etc. or parenterally, e.g., intravenously, in the form of sterile injectable solutions or suspensions. The enteral and parenteral compositions may be prepared by conventional means.

The DPP-IV inhibitor compound and its corresponding pharmaceutically acceptable acid addition salts, may be formulated into enteral and parenteral pharmaceutical compositions containing an amount of the active substance that is effective for treating conditions mediated by DPP-IV inhibition, such compositions in unit dosage form and such compositions comprising a pharmaceutically acceptable carrier.

The DPP-IV inhibitor compound, may be administered in enantiomerically pure form, e.g., >98%, preferably >99%; or together with the R enantiomer, e.g., in racemic form. The above dosage ranges are based on the compounds of formula (I), excluding the amount of the R enantiomer.

In view of their ability to inhibit DPP-IV, the DPP-IV inhibitor compound and its corresponding pharmaceutically acceptable acid addition salts, is useful in treating conditions mediated by DPP-IV inhibition. Based on the above and findings in the literature, it is expected that the compound disclosed herein is useful in the treatment of conditions, such as non-insulin-dependent diabetes mellitus, arthritis, obesity, allograft transplantation and calcitonin-osteoporosis. In addition, based on the roles of glucagon-like peptides, such as GLP-1 and GLP-2, and their association with DPP-IV inhibition, it is expected that the compounds disclosed herein are useful for example, to produce a sedative or anxiolytic effect, or to attenuate post-surgical catabolic changes and hormonal responses to stress, or to reduce mortality and morbidity after myocardial infarction, or in the treatment of conditions related to the above effects which may be mediated by GLP-1 and/or GLP-2 levels.

More specifically, e.g., the DPP-IV inhibitor compound, and its corresponding pharmaceutically acceptable acid addition salts, improve early insulin response to an oral glucose challenge and, therefore, are useful in treating non-insulin-dependent diabetes mellitus.

The DPP-IV inhibitor compound, useful in this invention are hygroscopic, presents stability problems, and are not inherently compressible. Consequently, there is a need to provide a free-flowing and cohesive composition capable of being directly compressed into strong tablets with an acceptable in vitro dissolution profile. Tablets may be defined as solid dosage pharmaceutical forms containing drug substances with or without suitable fillers. They are produced by compression or compaction of a formulation containing the active ingredient and certain excipients selected to aid in the processing and to improve the properties of the product. Tablets may be coated or uncoated and are made from powdered, crystalline materials. They may include various diluents, binders, disintegrants, lubricants, glidants and in many cases, colorants. Excipients used are classified according to the function they perform. For example, a glidant may be used to improve the flow of powder blend in the hopper and into the tablet die.

There has been widespread use of tablets since the latter part of the 19^{th} century and the majority of pharmaceutical dosage forms are marketed as tablets. Major reasons of tablet popularity as a dosage form are simplicity, low cost and the speed of production. Other reasons include stability of drug product, convenience in packaging, shipping and dispensing. To the patient or consumer, tablets offer convenience of administration, ease of accurate dosage, compactness, portability, blandness of taste, ease of administration and elegant distinctive appearance.

Tablets may be plain, film or sugar coated bisected, embossed, layered or sustained-release. They can be made in a variety of sizes, shapes and colors. Tablets may be swallowed, chewed or dissolved in the buccal cavity or beneath the tongue. They may be dissolved in water for local or topical application. Sterile tablets are normally used for parenteral solutions and for implantation beneath the skin.

In addition to the active or therapeutic ingredients, tablets may contain a number of inert materials known as excipients. They may be classified according to the role they play in the final tablet. The primary composition includes a filler, binder, lubricant and glidant. Other excipients which give physical characteristics to the finished tablet are coloring agents, and flavors in the case of chewable tablets. Without excipients most drugs and pharmaceutical ingredients cannot be directly-compressed into tablets. This is primarily due to the poor flow and cohesive properties of most drugs. Typically, excipients are added to a formulation to impart good flow and compression characteristics to the material being compressed. Such properties are imparted to these excipients through pretreatment steps, such as wet granulation, slugging, spray drying spheronization or crystallization.

Lubricants are typically added to prevent the tableting materials from sticking to punches, minimize friction during tablet compression, and allow for removal of the compressed tablet from the die. Such lubricants are commonly included in the final tablet mix in amounts usually less than 1 % by weight.

In addition, tablets often contain diluents which are added to increase the bulk weight of the blend resulting in a practical size for compression. This is often necessary where the dose of the drug is relatively small.

Another commonly used class of excipients in tablets is binders. Binders are agents, which impart cohesive qualities to the powdered material. Commonly used binders include starch, and sugars, such as sucrose, glucose, dextrose and lactose.

Disintegrants are often included to ensure that the tablet has an acceptable rate of disintegration. Typical disintegrants include starch derivatives and salts of carboxymethylcellulose.

Other desirable characteristics of excipients include the following:
- High-compressibility to allow strong tablets to be made at low compression forces;
- Good flow properties that can improve the flow of other excipients in the formula; and
- Cohesiveness (to prevent tablet from crumbling during processing, shipping and handling).

There are three commercially important processes for making compressed tablets: wet granulation, direct compression and dry granulation (slugging or roller compaction). The method of preparation and type of excipients are selected to give the tablet formulation the desired physical characteristics that allow for the rapid compression of the tablets. After compression, the tablets must have a number of additional attributes, such as appearance, hardness, disintegrating ability and an acceptable dissolution profile. Choice of fillers and other excipients will depend on the chemical and physical properties of the drug, behavior of the mixture during processing and the properties of the final tablets. Preformulation studies are done to determine the chemical and physical compatibility of the active component with proposed excipients.

The properties of the drug, its dosage forms and the economics of the operation will determine selection of the best process for tableting. Generally, both wet granulation and direct compression are used in developing a tablet.

The dry granulation method may be used where one of the constituents, either the drug or the diluent, has sufficient cohesive properties to be tabletted. The method consists of blending, slugging the ingredients, dry screening, lubrication and compression.

The wet granulation method is used to convert a powder mixture into granules having suitable flow and cohesive properties for tableting. The procedure consists of mixing the powders in a suitable blender followed by adding the granulating solution under shear to the mixed powders to obtain a granulation. The damp mass is then screened through a suitable screen and dried by tray drying or fluidized bed drying. Alternately, the wet mass may be dried and passed through a mill. The overall process includes weighing, dry powder blending, wet granulating, drying, milling, blending lubrication and compression.

In general, powders do not have sufficient adhesive or cohesive properties to form hard, strong granules. A binder is usually required to bond the powder particles together due to the poor cohesive properties of most powders. Heat and moisture sensitive drugs cannot usually be manufactured using wet granulation. The large number of processing steps and processing time are problems due to high level manufacturing costs. Wet granulation has also been known to reduce the compressibility of some pharmaceutical excipients, such as microcrystalline cellulose.

Direct compression is regarded as a relatively quick process where the powdered materials are compressed directly without changing the physical and chemical properties of the drug. The active ingredient(s), direct compression excipients and other auxiliary substances, such as a glidant and lubricant are blended in a twin shell blender or similar low shear apparatus before being compressed into tablets. This type of mixing was believed to be essential in order to prepare "pharmaceutically acceptable" dosage forms. Some pharmaceutical scientists believe that the manner in which a lubricant is added to a formulation must be carefully controlled. Accordingly, lubricants are usually added to a granulation by gentle mixing. It is also believed that prolonged blending of a lubricant with a granulation can materially affect hardness and disintegration time for the resulting tablets. Excessive blending of lubricants with the granulate ingredients can cause water proofing of the granule and reduces tablet hardness or strength of the compressed tablet. For these reasons, high-shear mixing conditions have not been used to prepare direct compression dosage forms.

The advantages of direct compression include uniformity of blend, few manufacturing steps involved, i.e., the overall process involves weighing of powders, blending and compression, hence less cost; elimination of heat and moisture, prime particle dissociation and physical stability.

Pharmaceutical manufacturers would prefer to use direct compression techniques over wet or dry granulation methods because of quick processing time and cost advantages. However, direct compression is usually limited to those situations where the drug or active ingredient has physical characteristics required to form pharmaceutically acceptable tablets. However, one or more excipients must often be combined with the active ingredient before the direct-compression method can be used since many ingredients do not have the necessary properties. Since each excipient added to the formulation increases the tablet size of the final product, manufacturers are often limited to using the direct-compression method in formulations containing a low dose of the active ingredient per compressed tablet.

A solid dosage form containing a high-dose drug, i.e., the drug itself comprises a substantial portion of the total compressed tablet weight, could only be directly compressed if the drug itself has sufficient physical characteristics, e.g., cohesiveness, for the ingredients to be directly compressed.

For an example, the DPP-IV inhibitor is considered a high-dose drug. Most tablet formulations include a range of 70-85% by weight of DPP-IV inhibitor per tablet. This high-dose drug, combined with its rather poor physical characteristics for direct compression, has not permitted direct compression as a method to prepare the final tablet. In addition, the active ingredients have poor stability in presence of water, another factor militating against the use of the wet granulation method.

Another limitation of direct compression as a method of tablet manufacturing is the potential size of the compressed tablets. If the amount of active ingredient is high, a pharmaceutical formulator may choose to wet granulate the active ingredient with other excipients to attain an acceptable sized tablet with the desired amount of active ingredient. The amount of filler, binder or other excipients needed in wet granulation is less than that required for direct compression since the process of wet granulation contributes toward the desired physical properties of the tablet.

Hydroxypropyl methylcellulose has been utilized in the pharmaceutical industry as a direct compression excipient for solid dose forms. Hydroxypropyl methylcellulose is a processed cellulose and controls drug release from solid dosage forms.

Despite the advantages of the direct compression, such as reduced processing time and cost, wet granulation is widely-used in the industry to prepare solid dosage forms. Wet granulation is often preferred over direct compression because wet granulation has a greater chance of overcoming any problems associated with the physical characteristics of various ingredients in the formulation. This provides material which has the required flow and cohesive properties necessary to obtain an acceptable solid dosage form.

The popularity of wet granulation compared to direct compression is based on at least three advantages. First, wet granulation provides the material to be compressed with better wetting properties, particularly in the case of hydrophobic drug substances. The addition of hydrophilic excipients makes the surface of the hydrophobic drug more hydrophilic, reducing disintegration and dissolution problems. Second, the content uniformity of the solid dosage form is generally improved with wet granulation because all of the granules usually contain the same amount of drug. Lastly, the segregation of drug(s) from excipients is avoided.

Segregation could be a potential problem with direct compression. The size and shape of particles comprising the granulate to be compressed are optimized through the wet granulation process. This is because when a dry solid is wet granulated the binder "glues" particles together, so that they agglomerate into spherical granules.

In spite of the advantages afforded by wet granulation in general, due to the instability of the compounds in the presence of water, it is desirable to directly compress tablets containing high-dose DPP-IV inhibitor. There is a need in the industry for techniques and pharmaceutical excipients which will allow manufacturers to prepare high-dose DPP-IV inhibitor tablets by direct compression.

It is an object of the invention to provide a DPP-IV inhibitor formulation in the form of a free-flowing, cohesive tableting powder, capable of being directly compressed into a tablet.

It is a further object of the invention to provide a direct compressed DPP-IV inhibitor tablet in unit dosage form having an acceptable dissolution profile, as well as acceptable degrees of hardness and resistance to chipping, as well as a short disintegration time.

It is a further object of the invention to provide a process for preparing a compressed DPP-IV inhibitor tablet by direct compression in unit dosage form.

The present invention provides a direct tableting, free-flowing particulate DPP-IV inhibitor formulation in the form of a tableting powder, capable of being directly compressed into a tablet having adequate hardness, rapid disintegration time and an acceptable dissolution pattern.

In addition to the active ingredient, the tableting powder contains a number of inert materials known as excipients. They may be classified according to the role they play in the final tablet. The primary composition includes fillers, binders or diluents, lubricants, disintegrants and glidants. Other excipients which give physical characteristics to the finished tablet are coloring agents, and flavors in the case of chewable tablets. Typically, excipients are added to a formulation to impart good flow and compression characteristics to the material being compressed.

The preferred formulation of this invention comprises the following: the active ingredient which is the DPP-IV inhibitor compound, the binders or diluents which are microcrystalline cellulose and lactose, the disintegrant which is sodium starch glycolate and the lubricant which is magnesium stearate.

One, two, three or more diluents can be selected. Examples of pharmaceutically acceptable fillers and pharmaceutically acceptable diluents include, but are not limited to, confectioner's sugar, compressible sugar, dextrates, dextrin, dextrose, lactose, mannitol, microcrystalline cellulose, powdered cellulose, sorbitol, sucrose and talc. The filler and/or diluent, e.g., may be present in an amount from about 15% to about 40% by weight of the composition. The preferred diluents include microcrystalline cellulose which is manufactured by the controlled hydrolysis of alpha-cellulose, obtained as a pulp from fibrous plant materials, with dilute mineral acid solutions. Following hydrolysis, the hydrocellulose is purified by filtration and the aqueous slurry is spray dried to form dry, porous particles of a broad size distribution. Suitable microcrystalline cellulose will have an average particle size of from about 20 nm to about 200 nm. Microcrystalline cellulose is available from several suppliers. Suitable microcrystalline cellulose includes Avicel PH 101, Avicel PH 102, Avicel PH 103, Avicel PH 105 and Avicel PH 200, manufactured by FMC Corporation. Particularly preferred in the practice of this invention is Avicel PH 102, which has the smallest surface area and pore structure. Preferably the microcrystalline cellulose is present in a tablet formulation in an amount of from about 25% to about 70% by weight. Another preferred range of this material is from about 30% to about 35% by weight; yet another preferred range of from about 30% to about 32% by weight.

Another diluent is lactose. Preferably, the lactose is ground to have an average particle size of between about 50 µm and about 500 µm prior to formulating. The lactose is present in the tablet formulation in an amount of from about 5% to about 40% by weight, and can be from about 18% to about 35% by weight, and most preferred, can be from about 20% to about 25% by weight.

One, two, three or more disintegrants can be selected. Examples of pharmaceutically acceptable disintegrants include, but are not limited to, starches; clays; celluloses; alginates; gums; cross-linked polymers, e.g., cross-linked polyvinyl pyrrolidone, cross-linked calcium carboxymethylcellulose and cross-linked sodium carboxymethylcellulose; soy polysaccharides; and guar gum. The disintegrant, e.g., may be present in an amount from about 2% to about 20%, e.g., from about 5% to about 10%, e.g., about 7% about by weight of the composition. A disintegrant is also an optional but useful component of the tablet formulation. Disintegrants are included to ensure that the tablet has an acceptable rate of disintegration. Typical disintegrants include starch derivatives and salts of carboxymethylcellulose. Sodium starch glycolate is the preferred disintegrant for this formulation. Preferably the disintegrant is present in the tablet formulation in an amount of from about 0% to about 10% by weight, and can be from about 1% to about 4% by weight, and most preferred, can be from about 1.5% to about 2.5% by weight.

One, two, three or more lubricants can be selected. Examples of pharmaceutically acceptable lubricants and pharmaceutically acceptable glidants include, but are not limited to, colloidal silica, magnesium trisilicate, starches, talc, tribasic calcium phosphate, magnesium stearate, aluminum stearate, calcium stearate, magnesium carbonate, magnesium oxide, polyethylene glycol, powdered cellulose and microcrystalline cellulose. The lubricant, e.g., may be present in an amount from about 0.1% to about 5% by weight of the composition; whereas, the glidant, e.g., may be present in an amount from about 0.1 % to about 10% by weight. Lubricants are typically added to prevent the tableting materials from sticking to punches, minimize friction during tablet compression and allow for removal of the compressed tablet from the die. Such lubricants are commonly included in the final tablet mix in amounts usually less than 1% by weight. The lubricant component may be hydrophobic or hydrophilic. Examples of such lubricants include stearic acid, talc and magnesium stearate. Magnesium stearate reduces the friction between the die wall and tablet mix during the compression and ejection of the tablets. It helps prevent adhesion of tablets to the punches and dies. Magnesium stearate also aids in the flow of the powder in the hopper and into the die. It has a particle size range of 450-550 microns and a density range of 1.00-1.80 g/mL. It is stable and does not polymerize within the tableting mix. The preferred lubricant, magnesium stearate is also employed in the formulation. Preferably, the lubricant is present in the tablet formulation in an amount of from about 0.25% to about 6%; also preferred is a level of about 0.5% to about 4% by weight; and most preferably from about 0.1 % to about 2% by weight. Other possible lubricants include talc, polyethylene glycol, silica and hardened vegetable oils. In an optional embodiment of the invention, the lubricant is not present in the formulation, but is sprayed onto the dies or the punches rather than being added directly to the formulation.
Other conventional solid fillers or carriers, such as, cornstarch, calcium phosphate, calcium sulfate, calcium stearate, magnesium stearate, stearic acid, glyceryl mono- and distearate, sorbitol, mannitol, gelatin, natural or synthetic gums, such as carboxymethyl cellulose, methyl cellulose, alginate, dextran, acacia gum, karaya gum, locust bean gum, tragacanth and the like, diluents, binders, lubricants, disintegrators, coloring and flavoring agents could optionally be employed.

Examples of pharmaceutically acceptable binders include, but are not limited to, starches; celluloses and derivatives thereof, e.g., microcrystalline cellulose, hydroxypropyl cellulose hydroxylethyl cellulose and hydroxylpropylmethyl cellulose; sucrose; dextrose; corn syrup; polysaccharides; and gelatin. The binder, e.g., may be present in an amount from about 10% to about 40% by weight of the composition.

Additional examples of useful excipients are described in the Handbook of pharmaceutical excipients, 3rd edition , Edited by A.H.Kibbe, Published by: American Pharmaceutical Association, Washington DC, ISBN: 0-917330-96-X, or Handbook of Pharmaceutical Excipients (4th edition), Edited by Raymond C Rowe - Publisher: Science and Practice.

Thus, in a first embodiment, the present invention concerns a pharmaceutical composition comprising;
(a) a DPP-IV inhibitor in free form or in acid addition salt form, namely LAF237;
(b) a pharmaceutically acceptable diluent,
   wherein in the unit dosage form, the weight of LAF237 on a dry weight basis to tablet weight of diluent ratio is of 0.5 to 0.25, preferably 0.4 to 0.28.
   Composition as described above, wherein at least one diluent is a microcrystalline cellulose and wherein in the unit dosage form, the weight of LAF237 on a dry weight basis to tablet weight of microcrystalline cellulose ratio is of 2 to 0.333, preferably 1 to 0.333, most preferably of 0.7 to 0.333.
   Composition as described above comprising between 20 and 120 mg of LAF237 preferably between 25 and 100m of LAF237 or a pharmaceutically acceptable acid addition salt thereof.
   Composition as described above wherein the diluent is selected from a microcrystalline cellulose and lactose, preferably microcrystalline cellulose and lactose are in the composition.
   Composition as described above which comprises in addition;
(c) 0-20% by weight on a dry weight basis of a pharmaceutically acceptable disintegrant;
(d) 0.1-10% by weight on a dry weight basis of a pharmaceutically acceptable lubricant.

Preferably composition as described above which comprises in addition;;
(c) 1-6% by weight on a dry weight basis of a pharmaceutically acceptable disintegrant;
(d) 0.25-6% by weight on a dry weight basis of a pharmaceutically acceptable lubricant.

The above ratios have been obtained on a dry weight basis for the DPP-IV inhibitor and diluents.

The unit dosage form, is any kind of pharmaceutical dosage form such as capsules, tablets, granules, chewable tablets, etc.

In a further, embodiment, the present invention concerns a pharmaceutical composition comprising;
(a) 5-60% by weight on a dry weight basis of a LAF 237 in free form or in acid addition salt form ;
(b) 40-95% by weight on a dry weight basis of a pharmaceutically acceptable diluent;
(c) 0-20% by weight on a dry weight basis of a pharmaceutically acceptable disintegrant; and optionally
(d) 0.1-10% by weight on a dry weight basis of a pharmaceutically acceptable lubricant.

Preferably the present invention concerns a pharmaceutical composition comprising;
(a) 20-40% by weight on a dry weight basis of LAF 237 in free form or in acid addition salt form ;
(b) 40-95% by weight on a dry weight basis of a pharmaceutically acceptable diluent;
(c) 0-10% by weight on a dry weight basis of a pharmaceutically acceptable disintegrant; and optionally
(d) 0.25-6% by weight on a dry weight basis of a pharmaceutically acceptable lubricant.

Most preferably the present invention concerns a pharmaceutical composition comprising;
(a) 20-35% by weight on a dry weight basis of a LAF 237 in free form or in acid addition salt form ;
(b) 40-95% by weight on a dry weight basis of a pharmaceutically acceptable diluent;
(c) 0-10% by weight on a dry weight basis of a pharmaceutically acceptable disintegrant; and optionally
(d) 0.25-6% by weight on a dry weight basis of a pharmaceutically acceptable lubricant.

Most preferably the present invention concerns a pharmaceutical composition comprising;
(a) 20-35% by weight on a dry weight basis of a LAF 237 in free form or in acid addition salt form ;
(b) 62-78% by weight on a dry weight basis of a pharmaceutically acceptable diluent;
(c) 0-10% by weight on a dry weight basis of a pharmaceutically acceptable disintegrant; and optionally
(d) 0.1-10% by weight on a dry weight basis of a pharmaceutically acceptable lubricant.

Most preferably the present invention concerns a pharmaceutical composition comprising;
(a) 20-35% by weight on a dry weight basis of a LAF 237 in free form or in acid addition salt form ;
(b) 62-78% by weight on a dry weight basis of a pharmaceutically acceptable diluent;
(c) 1-6% by weight on a dry weight basis of a pharmaceutically acceptable disintegrant; and optionally
(d) 0.25-6% by weight on a dry weight basis of a pharmaceutically acceptable lubricant.

Most preferably the present invention concerns a pharmaceutical composition comprising;
(a) 22-28% by weight on a dry weight basis of a LAF 237 in free form or in acid addition salt form ;
(b) 66-76% by weight on a dry weight basis of a pharmaceutically acceptable diluent;
(c) 0-6% by weight on a dry weight basis of a pharmaceutically acceptable disintegrant; and optionally
(d) 0.25-6% by weight on a dry weight basis of a pharmaceutically acceptable lubricant.

Most preferably the present invention concerns a pharmaceutical composition comprising;
(a) 22-28% by weight on a dry weight basis of a LAF 237 in free form or in acid addition salt form
(b) 66-76% by weight on a dry weight basis of a pharmaceutically acceptable diluent;
(c) 1-6% by weight on a dry weight basis of a pharmaceutically acceptable disintegrant; and optionally
(d) 0.25-6% by weight on a dry weight basis of a pharmaceutically acceptable lubricant.

In the present application the reference to a pharmaceutically acceptable diluent means at least one diluent, a mixture of e.g. 2 or 3 diluents is also covered.

Preferably the above described compositions comprise;
i) one or two diluents selected from microcrystalline cellulose and lactose
ii) the two diluents microcrystalline cellulose and lactose,
iii) 25-70% preferably 35-55% by weight on a dry weight basis of a pharmaceutically acceptable microcrystalline cellulose, or
iv) 25-70% preferably 35-55% by weight on a dry weight basis of a pharmaceutically acceptable microcrystalline cellulose and 5-40% preferably 18-35% of lactose.

Most preferably the above described compositions comprise one or two diluents selected from microcrystalline cellulose such as Avicel PH 102 and lactose.

Most preferably the pharmaceutical composition comprises the pharmaceutically acceptable lubricant (d).

In the present application the reference to a pharmaceutically acceptable disintegrant means at least one disintegrant, a mixture of e.g. 2 or 3 disintegrants is also covered.

In the present application the reference to a pharmaceutically acceptable lubricant means at least one lubricant, a mixture of e.g. 2 or 3 lubricants is also covered.

DPP-IV inhibitor is LAF237, preferred diluents are microcrystalline cellulose or lactose or preferably a combination of microcrystalline cellulose and lactose, preferred disintegrant is sodium starch glycolate, and preferred lubricant is magnesium stearate.

The particular components in the preferred composition are the following:
(a) 20-35% by weight on a dry weight basis of LAF237;
(b) 25-70% by weight on a dry weight basis of a pharmaceutically acceptable microcrystalline cellulose;
(c) 5-40% by weight on a dry weight basis of a pharmaceutically acceptable lactose;
(d) 0-10% by weight on a dry weight basis of a pharmaceutically acceptable sodium starch glycolate;
(e) 0.25-6% by weight on a dry weight basis of magnesium stearate.

The particular components in the preferred composition are the following:
(a) 25-35% by weight on a dry weight basis of LAF237;
(b) 25-70% by weight on a dry weight basis of a pharmaceutically acceptable microcrystalline cellulose;
(c) 5-40% by weight on a dry weight basis of a pharmaceutically acceptable lactose;
(d) 0-10% by weight on a dry weight basis of a pharmaceutically acceptable sodium starch glycolate;
(e) 0.25-6% by weight on a dry weight basis of magnesium stearate.

Another preferred composition is the following:
(a) 20-35% preferably 22-28% by weight on a dry weight basis of LAF237;
(b) 35-55% by weight on a dry weight basis of a pharmaceutically acceptable microcrystalline cellulose;
(c) 18-35% by weight on a dry weight basis of a pharmaceutically acceptable lactose;
(d) 1-4% by weight on a dry weight basis of a pharmaceutically acceptable sodium starch glycolate; and
(e) 0.5-4% by weight on a dry weight basis of magnesium stearate.

Still another preferred composition is the following:
(a) 30-35% by weight on a dry weight basis of LAF237;
(b) 35-50% by weight on a dry weight basis of a pharmaceutically acceptable microcrystalline cellulose;
(c) 18-35% by weight on a dry weight basis of a pharmaceutically acceptable lactose;
(d) 1-4% by weight on a dry weight basis of a pharmaceutically acceptable sodium starch glycolate; and
(e) 0.5-4% by weight on a dry weight basis of magnesium stearate.

In a further embodiment, the present invention concerns any one of the above described compositions wherein the pharmaceutically acceptable lubricant (d) is only optionally comprised in the formulation. But preferably the pharmaceutically acceptable lubricant (d) is comprised in the composition.

Preferably for compressed tablets especially for direct compressed tablets, the above described compositions comprise between 20 and 35% most preferably between 22 and 28% by weight on a dry weight basis of LAF237, in free form or in acid addition salt form.

Additional conventional excipients can optionally be added to the herein described formulations such as the conventional solid fillers or carriers described hereinabove.

The above described formulations are particularly adapted for the production of pharmaceutical tablets e.g compressed tablets or preferably direct compressed tablets, caplets or capsules and provides the necessary physical characteristics, dissolution and drug release profiles as required by one of ordinary necessary physical skill in the art. Therefore in an additional embodiment, the present invention concerns the use of any of the above described formulations, for the manufacture of pharmaceutical tablets, caplets or capsules in particular for granulation, direct compression and dry granulation (slugging or roller compaction).

The above formulations are also particularly useful for the production of tablets especially compressed tablets and very preferably direct compressed tablets.

In particular the tablets obtained with the above described formulations especially when processed in the form of direct compressed tablets or the below described direct compressed tablets, have very low friability problems, very good breaking strength, improved manufacturing robustness, optimal tablet thickness to tablet weight ratios (direct compressed tablets), less water in the formulation especially directed compressed tablet, good Dispersion Disintegration time DT according to the British Pharmacopoeia 1988, good Dispersion Quality.

This present invention of direct compression of DPP-IV inhibitor involves blending and compression. The choice of grades of excipients took into consideration particle size maintained within a range that allows homogeneity of the powder mix and content uniformity of DPP-IV inhibitor. It prevents segregation of powders in the hopper during direct compression. The advantages of using these excipients are that they impart compressibility, cohesiveness and flowability of the powder blend. In addition, the use of direct compression provides competitive unit production cost, shelf life, eliminates heat and moisture, allows for prime particle dissociation, physical stability and ensures particle size uniformity.

The described advantages of the claimed compositions are also very useful for e.g. roller compaction or wet granulation or to fill capsules.

In the development of the herein described pharmaceutical compositions, the applicant has discovered that the compressed tablets especially direct compressed tablet is particularly advantageous if;
i) the particles comprising the DPP-IV inhibitor have a particle size distribution of less than 250 µm preferably between 10 to 250 µm, and/or
ii) the water content of the tablet at less than 10% after 1 week at 25°C and 60% room humidity (RH), and/or
iii) tablet thickness to tablet weight ratios is of 0.002 to 0.06 mm/mg.

Thus, the present invention concerns a compressed pharmaceutical tablet preferably a direct compressed pharmaceutical tablet, comprising a DPP-IV inhibitor, in free form or in acid addition salt form, having physical properties that render the tableting into direct compressed pharmaceutical tablet unlikely or very difficult. The DPP-IV inhibitor is LAF237.

Thus in a first embodiment (a), the present invention concerns compressed tablets preferably direct compressed pharmaceutical tablets, wherein the dispersion contains particles comprising LAF237, in free form or in acid addition salt form, and wherein at least 60%, preferably 80% and most preferably 90% of the particle size distribution in the tablet is less than 250 µm or preferably between 10 to 250 µm.

The present invention concerns compressed tablets preferably direct compressed pharmaceutical tablets, wherein the dispersion contains particles comprising LAF237, in free form or in acid addition salt form, and wherein at least 60%, preferably 80% and most preferably 90% of the particle size distribution in the tablet is greater than 10 µm.

The term "wherein at least 60%, preferably 80% and most preferably 90%" means at least 60%, preferably at least 80% and most preferably at least 90%.

The term "wherein at least at least 25%, preferably 35% and most preferably 45%" means at least 25%, preferably at least 35% and most preferably at least 45%.

In particular the present invention concerns compressed tablets preferably direct compressed pharmaceutical tablets, wherein the dispersion contains particles comprising LAF237, in free form or in acid addition salt form, and wherein at least 25%, preferably 35% and most preferably 45% of the particle size distribution in the tablet is between 50 to 150 µm.

In a second embodiment (b), this invention concerns a compressed tablet, preferably a direct compressed pharmaceutical tablet wherein the dispersion contains particles comprising LAF237, in free form or in acid addition salt form, and wherein tablet thickness to tablet weight ratios is of 0.002 to 0.06 mm/mg preferably of 0.01 to 0.03 mm/mg.

The combination of the above first and second embodiments (a) and (b), provide compressed tablets preferably direct compressed tablets with good compaction characteristics.

Thus this invention concerns also a compressed tablet, preferably a direct compressed tablet wherein the dispersion contains particles comprising LAF237, in free form or in acid addition salt form, and wherein;
i) at least 60%, preferably 80% and most preferably 90% of the particle size distribution in the tablet is less than 250 µm or preferably between 10 to 250 µm, and
ii) tablet thickness to tablet weight ratios is of 0.002 to 0.06 mm/mg or of 0.01 to 0.03 mm/mg
preferably wherein;
i) at least 25 %, preferably 35% and most preferably 45% of the particle size distribution in the tablet is between 50 to 150 µm, and
ii) tablet thickness to tablet weight ratios is of 0.002 to 0.06 mm/mg or of 0.01 to 0.03 mm/mg.

In a third embodiment, this invention concerns a compressed tablet preferably a direct compressed pharmaceutical tablet wherein the dispersion contains particles comprising LAF237, in free form or in acid addition salt form, and wherein;
i) at least 60%, preferably 80% and most preferably 90% of the particle size distribution in the tablet is less than 250 µm preferably between 10 to 250 µm,
ii) the water content of the tablet is less than 10% after 1 week at 25°C and 60% RH, and
iii) tablet thickness to tablet weight ratios is of 0.002 to 0.06 mm/mg.

Preferably this invention concerns a compressed tablet most preferably a direct compressed tablet wherein the dispersion contains particles comprising LAF237, in free form or in acid addition salt form, and wherein;
i) at least 25%, preferably 35% and most preferably 45% of the particle size distribution in the tablet is between 50 to 150 µm,
ii) the water content of the tablet is less than 10% after 1 week at 25°C and 60% RH, and
iii) tablet thickness to tablet weight ratios is of 0.002 to 0.06 mm/mg.

Preferably this invention concerns a compressed tablet most preferably a direct compressed tablet wherein the dispersion contains particles comprising LAF237, in free form or in acid addition salt form, and wherein;
i) at least 25%, preferably 35% and most preferably 45% of the particle size distribution in the tablet is between 50 to 150 µm,
ii) the water content of the tablet is less than 5% after 1 week at 25°C and 60% RH, and
iii) tablet thickness to tablet weight ratios is of 0.002 to 0.06 mm/mg.

Preferably this invention concerns a compressed tablet, most preferably a direct compressed tablet wherein the dispersion contains particles comprising LAF237, in free form or in acid addition salt form, and wherein;
i) at least 25%, preferably 35% and most preferably 45% of the particle size distribution in the tablet is between 50 to 150 µm,
ii) the water content of the tablet is less than 5% after 1 week at 25°C and 60% RH, and
iii) tablet thickness to tablet weight ratios is of 0.01 to 0.03 mm/mg

In a very preferred aspect, the above described three embodiments i.e. compressed tablets and direct compressed tablets contain the herein described compositions such as a pharmaceutical composition comprising;
(a) 20-35% by weight on a dry weight basis of LAF237;
(b) 40-95% by weight on a dry weight basis of a pharmaceutically acceptable diluent;
(c) 0-10% by weight on a dry weight basis of a pharmaceutically acceptable disintegrant;
(d) 0.25-6% by weight on a dry weight basis of a pharmaceutically acceptable lubricant.

Preferably the LAF237 particles comprise more than 70% of DPPIV inhibitor, most preferably more than 90% or 95% and even more preferably more than 98% of DPPIV inhibitor.

Preferably the LAF237 particles comprise more than 70% of LAF237, most preferably more than 90% or 95% and even more preferably more than 98% of LAF237.

It has been discovered that the selected particle size distribution of LAF237 were particularly important to provide the best compaction of the tablets.

In an additional preferred embodiment, the particle size distribution of the selected excipients (b), (c) and/or (d) is similar to the particle size distribution of LAF237 particles.

The term "similar", means that the particle size distribution of the excipient in the tablet is between 5 and 400 µm, or between 10 and 300 µm, preferably between 10 to 250 µm.

The preferred excipients with an adapted particle size distribution can be picked from e.g. Handbook of Pharmaceutical Excipients (4th edition), Edited by Raymond C Rowe - Publisher: Science and Practice.

Particle size of drug, LAF237 particles size, is controlled by crystallisazion, drying and/or milling/sieving (non limiting examples are described below). Particle size can also be comminuted using roller compaction and milling/sieving. Producing the right particle size is well known and described in the art such as in "Pharmaceutical dosage forms: volume 2, 2nd edition, Ed.: H.A.Lieberman, L.Lachman, J.B.Schwartz (Chapter 3: SIZE REDUCTION)".

Multiple particle sizes have been studied and it has been discovered that the herein described specific size range provides unexpected good results for direct compaction.

PARTICLE SIZE DISTRIBUTION ESTIMATION BY ANALYTICAL SIEVING: Particle size distribution is measured using Sieve analysis, Photon Correlation Spectroscopy or laser diffraction (international standart ISO 13320-1), or electronic sensing zone, light obstruction, sedimentation or microscopy which are procedures well known by the person skilled in the art. Sieving is one of the oldest methods of classifying powders by particle size distribution. Such methods are well known and described in the art such as in any analytical chemistry text book or by the United State Pharmacopeia's (USP) publication USP-NF (2004 - Chapter 786 - (The United States Pharmacopeial Convention, Inc., Rockville, MD)) which describes the US Food and Drug Administration (FDA) enforceable standards. The used techniques are e.g. described in Pharmaceutical dosage forms: volume 2, 2nd edition, Ed.: H.A.Lieberman, L.Lachman, J.B.Schwartz is a good example. It also mentions (page 187) additional methods: Electronic sensing zone, light obstruction, air permeation, sedimentation in gas or liquid.

In an air jet sieve measurement of particle size, air is drawn upwards, through a sieve, from a rotating slit so that material on the sieve is fluidised. At the same time a negative pressure is applied to the bottom of the sieve which removes fine particles to a collecting device. Size analyses and determination of average particle size are performed by removal of particles from the fine end of the size distribution by using single sieves consecutively. See also "Particle Size Measurement", 5th Ed. , p 178, vol. 1; T. Allen, Chapman & Hall, London, UK, 1997, for more details on this. For a person skilled in the art, the size measurement as such is thus of conventional character.

Water content of the tablet can be measured using Loss on drying method or Karl-Fischer method which are well known methods to the person skilled in the art (e.g. water content can be measured by loss on drying by thermogrametry). Such methods are well known and described in the art such as in any analytical chemistry text book (J.A. Dean, Analytical Chemistry Handbook, Section 19, McGraw-Hill, New York, 1995) or by the United State Pharmacopeia's (USP) publication USP-NF (2004) which describes the US Food and Drug Administration (FDA) enforceable standards ((2004 - USP - Chapter 921).

Tablet thickness is measurable using a ruler, vernier caliper, a screw gauge or any electronic method to measure dimensions. We take the tablet thickness in mm and divide by tablet weight in mg to get the ratio. Such methods are well known and described in the art such as in any analytical chemistry text book or by the United State Pharmacopeia's (USP) publication USP-NF (2004) which describes the US Food and Drug Administration (FDA) enforceable standards.

This invention provides in particular a compressed tablet or direct compressed tablet which is capable of dispersing in water within a period of 5 to 15 minutes to provide a dispersion which is capable of passing through a sieve screen with a mesh aperture of 710 µm in accordance with the herein defined British Pharmacopoeia test for dispersible tablets.

A tablet according to the invention, as well as being quickly dispersible in water, has the added advantage that it meets the British Pharmacopoeia (B.P.) test for dispersible tablets in respect of dispersion times and dispersion quality (i.e. passage through a 710 µm sieve).

Preferably the dispersion time of a tablet according to the invention is less than 15 minutes, more preferably less than 12 minutes and most preferably less than 10 minute.

A further advantage of the tablets according to invention is that because a relatively fine dispersion is formed the tablet will have a lower dissolution time and thus the drug may be absorbed into the blood stream much faster. Furthermore the fast dispersion times and relatively fine dispersions obtained with tablets according to the invention are also advantageous for swallowable tablets. Thus tablets according to the invention can be presented both for dispersion in water and also for directly swallowing. Those tablets according to the invention that are intended for swelling are preferably film-coated to aid swallowing.

In a further embodiment the present invention concerns a compressed tablet with improved dissolution rates (dissolution of the drug), wherein the dispersion contains LAF237 particles comprising LAF237, in free form or in acid addition salt form, wherein at least 60%, preferably 80% and most preferably 90% of the particle size distribution in the tablet is between 10 to 250 mm,
and wherein
i) between 0 and 10 minutes 85 to 99.5 % of the active ingredient is released, and
ii) between 10 and 15 minutes 90 to 99.5 % of the active ingredient is released,
preferably wherein,
i) between 0 and 10 minutes 88 to 99.5 % of the active ingredient is released, and
ii) between 10 and 15 minutes 95 to 99.5 % of the active ingredient is released,
or preferably
i) between 0 and 10 minutes 89 to 94 % of the active ingredient is released, and
ii) between 10 and 15 minutes 96 to 99 % of the active ingredient is released

The Paddle method to measure the drug dissolution rate (% of release) is used with 1000ml of 0.01 N HCl. Such methods are well known and described in the art such as in any analytical chemistry text book or by the United State Pharmacopeia's (USP) publication USP-NF (2004 - Chapter 711) which describes the US Food and Drug Administration (FDA) enforceable standards.

The invention also provides a process for preparing a compressed DPP-IV inhibitor tablet in unit dosage form, wherein;
i) at least 60%, preferably 80% and most preferably 90% of the particles comprising LAF237, in free form or in acid addition salt form, in the tablet have a particle size distribution of between 10 to 250 µm,
ii) the water content of the tablet is less than 10% after 1 week at 25°C and 60% RH, and
iii) tablet thickness to tablet weight ratios is of 0.002 to 0.06 mm
which comprises:
(a) blending as a % by weight on a dry weight basis:
   (i) 5-60% by weight on a dry weight basis of LAF237; and
   (ii) and at least one excipient selected from a diluent, a disintegrant and a lubricant,
   to form a DPP-IV inhibitor formulation in the form of a tableting powder, capable of being directly compressed into a tablet; and
(b) compressing the formulation prepared during step (a) to form the compressed DPP-IV inhibitor tablet in unit dosage form.

Preferably the above described process comprises:
(a) blending as a % by weight on a dry weight basis:
   (i) 5-60% by weight, on a dry weight basis of LAF237;
   (ii) 40-95% by weight on a dry weight basis of a pharmaceutically acceptable diluent;
   (iii) 0-20% by weight on a dry weight basis of a pharmaceutically acceptable disintegrant; and
   (iv) 0.1-10% by weight on a dry weight basis of a pharmaceutically acceptable lubricant,
   to form a DPP-IV inhibitor formulation in the form of a tableting powder, capable of being directly compressed into a tablet; and
(b) compressing the formulation prepared during step (a) to form the compressed DPP-IV inhibitor tablet in unit dosage form.

Most preferably the process comprises:
(a) blending as a % by weight on a dry weight basis:
   (i) 25-35% by weight on a dry weight basis of LAF237;
   (ii) 40-95% by weight on a dry weight basis of a pharmaceutically acceptable diluent;
   (iii) 0-10% by weight on a dry weight basis of a pharmaceutically acceptable disintegrant; and
   (iv) 0.25-6% by weight on a dry weight basis of a pharmaceutically acceptable lubricant,
   to form a DPP-IV inhibitor formulation in the form of a tableting powder, capable of being directly compressed into a tablet; and
(b) compressing the formulation prepared during step (a) to form the compressed DPP-IV inhibitor tablet in unit dosage form.

Preferably the blended composition used in step (a) is selected from the herein described preferred formulations.

The DPP IV inhibitor is LAF237, preferred diluents are microcrystalline cellulose or lactose or preferably a combination of microcrystalline cellulose and lactose, preferred disintegrant is sodium starch glycolate, and preferred lubricant is magnesium stearate.

In a best embodiment the process comprises:
(a) blending as a % by weight on a dry weight basis:
   (i) 20-35% or preferably 25-30% by weight by weight on a dry weight basis of LAF237, in free form or in acid addition salt form;
   (ii) 25-70% by weight or preferably 35-50% by weight on a dry weight basis of a pharmaceutically acceptable microcrystalline cellulose such as Avicel PH 102;
   (iii) 5-40% by weight or preferably 18-35% by weight on a dry weight basis of a pharmaceutically acceptable lactose;
   (iv) 0-10% by weight or preferably 1-4% by weight on a dry weight basis of a pharmaceutically acceptable sodium starch glycolate; and
   (v) 0.25- 6% by weight or preferably 0.5-4% by weight on a dry weight basis of a pharmaceutically acceptable magnesium stearate. to form a DPP-IV inhibitor formulation in the form of a tableting powder, capable of being directly compressed into a tablet; and
(b) compressing the formulation prepared during step (a) to form the compressed DPP-IV inhibitor tablet in unit dosage form.

The invention also provides a process for preparing a compressed DPP-IV inhibitor tablet in unit dosage form which comprises:
(a) blending as a % by weight on a dry weight basis:
   (i) 30-32% by weight on a dry weight basis of LAF237, in free form or in acid addition salt form;
   (ii) 40-45% by weight on a dry weight basis of a pharmaceutically acceptable microcrystalline cellulose (Avicel PH 102);
   (iii) 20-25% by weight on a dry weight basis of a pharmaceutically acceptable lactose;
   (iv) 1.5-2% by weight on a dry weight basis of a pharmaceutically acceptable sodium starch glycolate; and
   (v) 0.1-2% by weight on a dry weight basis of magnesium stearate,
   to form a DPP-IV inhibitor formulation in the form of a tableting powder, capable of being directly compressed into a tablet; and
(b) compressing the formulation prepared during step (a) to form the compressed DPP-IV inhibitor tablet in unit dosage form.

The invention also provides a process for preparing a compressed DPP-IV inhibitor tablet in unit dosage form which comprises:
(a) blending as a % by weight on a dry weight basis:
   (i) 23-28% by weight on a dry weight basis of LAF237, in free form or in acid addition salt form;
   (ii) 45-50% by weight on a dry weight basis of a pharmaceutically acceptable microcrystalline cellulose (Avicel PH 102);
   (iii) 20-25% by weight on a dry weight basis of a pharmaceutically acceptable lactose;
   (iv) 1.5-2% by weight on a dry weight basis of a pharmaceutically acceptable sodium starch glycolate; and
   (v) 0.1-2% by weight on a dry weight basis of magnesium stearate,
   to form a DPP-IV inhibitor formulation in the form of a tableting powder, capable of being directly compressed into a tablet; and
(b) compressing the formulation prepared during step (a) to form the compressed DPP-IV inhibitor tablet in unit dosage form.

Before the compression step (b) a sieving step is preferably applied to the formulation for basic delumping i.e. to get rid of any agglomerates/cakes.

The final product is prepared in the form of tablets, by employing conventional tableting or similar machinery.

The DPP-IV inhibitor is 1-[3-hydroxy-adamant-1-ylamino)-acetyl]-pyrrolidine-2(S)-carbonitrile (LAF237 or vildagliptin).

In a further aspect, the present invention concerns the use of the herein described formulations, capsules, tablets, compressed tables, direct compressed tablets for the treatment of conditions, such as non-insulin-dependent diabetes mellitus, arthritis, obesity, allograft transplantation, calcitonin-osteoporosis, Heart Failure, Impaired Glucose Metabolism), IGT (Impaired Glucose Tolerance), neurodegenerative diseases such as Alzheimer's and Parkinson disease, modulating hyperlipidemia, modulating conditions associated with hyperlipidemia or for lowering VLDL, LDL and Lp(a) levels, cardiovascular or renal diseases e.g. diabetic cardiomyopathy, left or right ventricular hypertrophy, hypertrophic medial thickening in arteries and/or in large vessels, mesenteric vasculature hypertrophy, mesanglial hypertrophy, neurodegenerative disorders and cognitive disorders, to produce a sedative or anxiolytic effect, to attenuate post-surgical catabolic changes and hormonal responses to stress, to reduce mortality and morbidity after myocardial infarction, the treatment of conditions related to the above effects which may be mediated by GLP-1 and/or GLP-2 levels.

In each case in particular in the compound claims, the final products of the working examples, the subject matter of the final products, the analytical and measurement methods (e.g. USP documents) the methods to obtain the right particles size, the pharmaceutical preparations, the excipients and the claims are hereby incorporated into the present application by reference to the herein mentioned publications or patent applications.

This invention is further illustrated by the following example:

### Example 1

To prepare the 25 mg tablet size (directly compressed tablet), a batch size of 7 kg is prepared using amounts corresponding to the following per unit: 25 mg per unit of the compound 1-[3-hydroxy-adamant-1-ylamino)-acetyl]-pyrrolidine-2(S)-carbonitrile is mixed with 35.1 mg of microcrystalline cellulose, 17.5 mg anhydrous lactose and 1.6 mg sodium starch glycolate. The ingredients are pre-blended together in a commercial bin blender, then sieved through a 500 µm or 850 µm screen. The mix is blended again in the bin blender, then the necessary amount of the magnesium stearate to yield the 0.8 mg magnesium stearate per 25 mg tablet size, is added. Blending in each step is conducted at about 150-450 rotations, to ensure homogeneity of the mixture. Following blending again in the bin blender, the mix can be tabletted in a conventional tableting machine. The individual tablet weight for the 25 mg tablet is 80 mg. Tablets having 50 mg active ingredient weigh 160 mg, and 100 mg active ingredient tablets weigh 320 mg, respectively. The blend is a powder which has excellent compressibility into the desired tablet size.

### Example 2

The same process as described above in example 1, can be applied to produce the below described preferred 50 mg tablet (directly compressed).

| **Components** | **Composition per unit (mg)** | **Quantity per batch (kg)** |
|---|---|---|
| LAF 237 drug substance | 50.00 | 65.0 |
| Microcrystalline cellulose, PH102 (Ph.Eur., NF) | 95.68 | 124.38 |
| Lactose anhydrous DT (USP, Ph.Eur.) | 47.82 | 62.17 |
| Sodium starch glycolate (USP, Ph.Eur.) | 4.00 | 5.2 |
| Magnesium stearate (Ph.Eur, NF) | 2.50 | 3.25 |
| Total weight, per tablet or per batch | 200.0 | 260.0 |

### Example 3: The tablets prepared in accordance with the above Description and examples can be tested as follows.

### Tablet Evaluation Methods

1. Average tablet weight. Twenty tablets are weighed on an analytical balance and the average tablet weight calculated.
2. Tablet breaking strength (kilo bond-kp). 5 tablets are individually tested using a Schleuniger crushing strength tester, and the average breaking strength calculated.
3. Friability (% loss). 10 tablets, accurately weighed, are subjected to 10 minutes friability testing using a Roche Friabilator. The tablets are dedusted, reweighed, and the weight loss due to the friability is calculated as a percentage of the initial weight.
4. Dispersion Disintegration time DT (The test for dispersible tablets defined in the British Pharmacopoeia, 1988, Volume II, page 895 - BP 1988). 6 tablets are tested in accordance to the above-defined BP test (without discs) for dispersible tablets. This utilizes water at a temperature of 19°- 21° C.
5. Dispersion Quality. In accordance with the BP uniformity of dispersion test for dispersible tablets (BP 1988 Volume II page 895), two tablets are placed in 100 ml of water at 19°-21° C. and allowed to disperse.

### Granule Evaluation Methods

1. Loss on Drying (LOD). The residual moisture content of the granule (LOD) can be determined on a 3-4 g sample using a Computrac moisture analyser set at 90° C. operated in accordance with the manufacturer's procedure.
2. Weight Median Diameter (WMD). A 10 g sample of granule is sifted for 2 minutes at suitable pulse and sift amplitudes in an Allen Bradley sonic sifter in accordance with manufacturer's instructions. Sieves of 300 µm, 250 µm, 200 µm, 150 µm, 100 µm, 53 µm and 40 µm are used. The WMD is calculated from the cumulative percentage undersize size distribution using a computer program.

### Example 4:

### Improved manufacturing robustness

A preliminary compactibility assessment is carried out on a Carver press using different formulations as well as binary mixtures of LAF 237 with different excipients e.g. microcrystalline cellulose (Avicel PH102).

Data demonstrate that our claimed compositions on being compressed with increasing levels of pressure (compression force) show a substantially useful increase in tablet strength. In particular e.g. mixture of LAF237 and Avicel show a substantially useful increase in tablet strength. These results indicated that from compactibility point of view microcrystalline cellulose e.g. Avicel would a preferred excipient to be combined with LAF237. With increasing pressure (compression force) our claimed formulations and selected ranges show a substantially useful increase in tablet strength.

A compactibility study (D. Becker, personal communication) is carried out on an instrumented Korsch single station press with force and displacement sensors on both upper and lower punches.

A clear indication is afforded from these data that LAF237 tablets are very likely to have poor tablet hardness/crushing strength unless diluted out using sufficient filler with excellent compactibility. Our claimed formulations and selected ranges are particularly adapted to provide the required compactibility. Microcrystalline cellulose e.g. Avicel is a good choice for a filler in this respect.

### Example 5: Friability

Evaluation is carried out using a Manesty Betapress at 6 different settings: strain rate settings of 66-90 rpm (63,000-86,000 TPH) and force of 7.5-15 kN. The trials uses Flat-faced Beveled-edge (FFBE) tooling of 9 mm diameter for 250 mg tablets and 10 mm diameter for 310 mg tablets (other diameters are used depending on the weight of the tested tablet) . Total tablet weights were selected so that both the 9 and 10 mm FFBE tablets would have 100 mg of LAF237 and identical tablet thickness. Friability, Compression profile, Strain rate profile and Weight variation are the measured outcomes. Study design and the friability results obtained from the study are used to determine the variables (particle size distribution in the formulation, tablet weight, tablet thickness and weight, water content in the tablet etc) impacting the outcome of hardness.

### Example 6: Mechanical stress (particle size distribution)

The material in the desired particle size range can be produced from any form of vildagliptin e.g. amorphous vildagliptin, by mechanical stress. This stress can be mediated by impact, shear or compression. In most commercially available grinding equipment a combination of these principles occurs. For vildagliptin preferably a mechanical impact or jet mill is used.

The most preferable mechanical impact mill can be equipped with different kind of beaters, screens, liners or with pin plates. For our process preferably an impact mill with plate beater and a slit screen 5 * 2.5 cm is used. The impact speed should be variable between 20 and 100 m/s (as peripheral speed) to adapt to any batch to batch variation. In our case a peripheral speed of the beater of about 40 - 50 m/s is used.

## Claims

1. A compressed pharmaceutical tablet or a direct compressed pharmaceutical tablet, wherein the dispersion contains particles comprising a DPP-IV inhibitor which is (S)-1-[(3-hydroxy-1-adamantyl)amino]acetyl-2-cyano-pyrrolidine in free form or in acid addition salt form, and wherein at least 60% of the particle size distribution in the tablet is less than 250 µm.

2. A compressed pharmaceutical tablet or a direct compressed pharmaceutical tablet according to claim 1, wherein the dispersion contains particles comprising DPP-IV inhibitor which is (S)-1-[(3-hydroxy-1-adamantyl)amino]acetyl-2-cyano-pyrrolidine in free form or in acid addition salt form, and wherein;
i) at least 60% of the particle size distribution in the tablet is less than 250 µm preferably between 10 to 250 µm, and
ii) tablet thickness to tablet weight ratios is of 0.002 to 0.06 mm/mg or of 0.01 to 0.03 mm/mg

3. A compressed pharmaceutical tablet or a direct compressed pharmaceutical tablet according to claim 1 or 2, wherein the dispersion contains particles comprising DPP-IV inhibitor which is (S)-1-[(3-hydroxy-1-adamantyl)amino]acetyl-2-cyano-pyrrolidine in free form or in acid addition salt form, and wherein;
i) at least 60% of the particle size distribution in the tablet is less than 250 µm preferably between 10 to 250 µm,
ii) the water content of the tablet is less than 10% after 1 week at 25°C and 60% RH, and
iii) tablet thickness to tablet weight ratios is of 0.002 to 0.06 mm/mg.

4. A compressed pharmaceutical tablet or a direct compressed pharmaceutical tablet according to any one of claims 1 to 3, wherein the particle size distribution in the tablet is between 50 to 150 µm.

5. A compressed pharmaceutical tablet or a direct compressed pharmaceutical tablet according to any one of claims 1 to 4, wherein the water content of the tablet is less than 5% after 1 week at 25°C and 60% RH

6. A compressed pharmaceutical tablet or a direct compressed pharmaceutical tablet according to any one of claims 1 to 5, wherein tablet thickness to tablet weight ratios is of 0.01 to 0.03 mm/mg

7. A compressed pharmaceutical tablet or a direct compressed pharmaceutical tablet according to any one of claims 1 to 6, wherein at least 60% or at least 80% of the particle size distribution in the tablet is between 10 to 250 µm.

8. A compressed pharmaceutical tablet or a direct compressed pharmaceutical tablet according to any one of claims 1 to 6, wherein at least 25% or at least 35% of the particle size distribution in the tablet is between 50 to 150 µm.

9. A compressed pharmaceutical tablet or a direct compressed pharmaceutical tablet according to any one of claims 1 to 8 wherein the tablet comprises
(a) 5-60% by weight on a dry weight basis of a DPP-IV inhibitor which is (S)-1-[(3-hydroxy-1-adamantyl)amino]acetyl-2-cyano-pyrrolidine in free form or in acid addition salt form;
(b) 40-95% by weight on a dry weight basis of a pharmaceutically acceptable diluent;
(c) 0-20% by weight on a dry weight basis of a pharmaceutically acceptable disintegrant; and optionally
(d) 0.1-10% by weight on a dry weight basis of a pharmaceutically acceptable lubricant.

10. A compressed pharmaceutical tablet or a direct compressed pharmaceutical tablet according to claim 9 wherein the tablet comprises
(a) 20-40% preferably 20-35% by weight on a dry weight basis of a DPP-IV inhibitor which is (S)-1-[(3-hydroxy-1-adamantyl)amino]acetyl-2-cyano-pyrrolidine in free form or in acid addition salt form;
(b) 40-95% by weight on a dry weight basis of a pharmaceutically acceptable diluent;
(c) 0-10% by weight on a dry weight basis of a pharmaceutically acceptable disintegrant; and optionally
(d) 0.25-6% by weight on a dry weight basis of a pharmaceutically acceptable lubricant.

11. A compressed pharmaceutical tablet or a direct compressed pharmaceutical tablet according to claims 9 to 10, wherein the tablet comprises
(a) 20-35% by weight on a dry weight basis of a (S)-1-[(3-hydroxy-1-adamantyl)amino]acetyl-2-cyano-pyrrolidine in free form or in acid addition salt form;
(b) 62-78% by weight on a dry weight basis of a pharmaceutically acceptable diluent;
(c) 0-10% by weight on a dry weight basis of a pharmaceutically acceptable disintegrant; and optionally
(d) 0.1-10% by weight on a dry weight basis of a pharmaceutically acceptable lubricant.

12. A compressed pharmaceutical tablet or a direct compressed pharmaceutical tablet according to claims 9 to 11, wherein the tablet comprises;
(a) 22-28% by weight on a dry weight basis of a (S)-1-[(3-hydroxy-1-adamantyl)amino]acetyl-2-cyano-pyrrolidine in free form or in acid addition salt form.

13. A compressed pharmaceutical tablet or a direct compressed pharmaceutical tablet according to any one of claims 9 to 10 comprising;
(a) 30-35 % by weight on a dry weight basis of a (S)-1-[(3-hydroxy-1-adamantyl)amino]acetyl-2-cyano-pyrrolidine in free form or in acid addition salt form, and
(b) 58-72% by weight on a dry weight basis of a pharmaceutically acceptable diluent;

14. A compressed pharmaceutical tablet or a direct compressed pharmaceutical tablet according to any one of claims 9 to 13 comprising;
i) one or two diluents selected from microcrystalline cellulose and lactose
ii) the two diluents microcrystalline cellulose and lactose,
iii) 25-70% preferably 35-55% by weight on a dry weight basis of a pharmaceutically acceptable microcrystalline cellulose, or
iv) 25-70% preferably 35-55% by weight on a dry weight basis of a pharmaceutically acceptable microcrystalline cellulose and 5-40% preferably 18-35% by weight on a dry weight basis of lactose.

15. A compressed pharmaceutical tablet or a direct compressed pharmaceutical tablet according to any one of claims 9 to 14 comprising;
(c) 1-6% by weight on a dry weight basis of a pharmaceutically acceptable disintegrant, and/or
(d) 0.1-10% by weight on a dry weight basis of a pharmaceutically acceptable lubricant.;

16. A compressed pharmaceutical tablet or a direct compressed pharmaceutical tablet according to any one of claims 9 to 13 comprising;
(a) 20-35% by weight on a dry weight basis of (S)-1-[(3-hydroxy-1-adamantyl)amino]acetyl-2-cyano-pyrrolidine in free form or in acid addition salt form;
(b) 25-70% by weight on a dry weight basis of a pharmaceutically acceptable microcrystalline cellulose;
(c) 5-40% by weight on a dry weight basis of a pharmaceutically acceptable lactose;
(d) 0-10% by weight on a dry weight basis of a pharmaceutically acceptable sodium starch glycolate;
(e) 0.25-6% by weight on a dry weight basis of magnesium stearate.

17. A compressed pharmaceutical tablet or a direct compressed pharmaceutical tablet according to any one of claims 9 to 13 comprising;
(a) 30-35% preferably 30-32% by weight on a dry weight basis of (S)-1-[(3-hydroxy-1-adamantyl)amino]acetyl-2-cyano-pyrrolidine in free form or in acid addition salt form;
(b) 35-50% preferably 40-45% by weight on a dry weight basis of a pharmaceutically acceptable microcrystalline cellulose;
(c) 18-35% preferably 20-25% by weight on a dry weight basis of a pharmaceutically acceptable lactose;
(d) 1-4% preferably 1.5-2.5% by weight on a dry weight basis of a pharmaceutically acceptable sodium starch glycolate; and
(e) 0.5-4% preferably 0.1-2% by weight on a dry weight basis of magnesium stearate.

18. A compressed pharmaceutical tablet or a direct compressed pharmaceutical tablet according to any one of claims 9 to 13 comprising;
(a) 20-35% preferably 22-28% by weight on a dry weight basis of of (S)-1-[(3-hydroxy-1-adamantyl)amino]acetyl-2-cyano-pyrrolidine in free form or in acid addition salt form;
(b) 35-55% by weight on a dry weight basis of a pharmaceutically acceptable microcrystalline cellulose;
(c) 18-35% by weight on a dry weight basis of a pharmaceutically acceptable lactose;
(d) 1-4% by weight on a dry weight basis of a pharmaceutically acceptable sodium starch glycolate; and
(e) 0.5-4% by weight on a dry weight basis of magnesium stearate.

19. A compressed pharmaceutical tablet or a direct compressed pharmaceutical tablet according to any one of claims 9 to 13 comprising;
(a) from about 22% to about 28% by weight on a dry weight basis of (S)-1-[(3-hydroxy-1-adamantyl)amino]acetyl-2-cyano-pyrrolidine in free form or in acid addition salt form;
(b) from about 45% to about 50% by weight on a dry weight basis of a pharmaceutically acceptable microcrystalline cellulose;
(c) from about 20% to about 25% by weight on a dry weight basis of a pharmaceutically acceptable lactose;
(d) from about 1.5% to about 2.5% by weight on a dry weight basis of a pharmaceutically acceptable sodium starch glycolate; and
(e) from about 0.1% to about 2% by weight on a dry weight basis of magnesium stearate.

20. A compressed pharmaceutical tablet or a direct compressed pharmaceutical tablet according to any one of claims 1 to 19, wherein
i) between 0 and 10 minutes 85 to 99.5 % of the active ingredient is released, and
ii) between 10 and 15 minutes 90 to 99.5 % of the active ingredient is released.

21. A compressed pharmaceutical tablet or a direct compressed pharmaceutical tablet according to any one of claims 1 to 19, wherein the particle size distribution of the pharmaceutical excipients in the tablet is between 5 and 400 µm.

22. A compressed pharmaceutical tablet according to any one of claims 1 to 21, which is a direct compressed tablet.

23. Process for preparing a direct compressed tablet according to any one of claims 1 to 22, in unit dosage form, which comprises:
(a) blending as a % by weight on a dry weight basis:
(i) 6-60% by weight on a dry weight basis of DPP-IV inhibitor; and
(ii) and at least one excipient selected from a diluent, a disintegrant and a lubricant,
to form a DPP-IV inhibitor formulation in the form of a tableting powder, capable of being directly compressed into a tablet; and
(b) compressing the formulation prepared during step (a) to form the compressed DPP-IV inhibitor tablet in unit dosage form
said DPP-IV inhibitor being (S)-1-[(3-hydroxy-1-adamantyl)amino]acetyl-2-cyano-pyrrolidine in free form or in acid addition salt form.

24. Process for preparing a direct compressed tablet according to any one of claims 1 to 22, in unit dosage form, which comprises:
(a) blending as a % by weight on a dry weight basis:
(i) 25-35% by weight on a dry weight basis of DPP-IV inhibitor;
(ii) 40-95% by weight on a dry weight basis of a pharmaceutically acceptable diluent;
(iii) 0-10% by weight on a dry weight basis of a pharmaceutically acceptable disintegrant; and
(iv) 0.25-6% by weight on a dry weight basis of a pharmaceutically acceptable lubricant,
to form a DPP-IV inhibitor formulation in the form of a tableting powder, capable of being directly compressed into a tablet; and
(b) compressing the formulation prepared during step (a) to form the compressed DPP-IV inhibitor tablet in unit dosage form,
said DPP-IV inhibitor being (S)-1-[(3-hydroxy-1-adamantyl)amino]acetyl-2-cyano-pyrrolidine in free form or in acid addition salt form.

25. Process according to claim 24 wherein the blended formulation comprises:
(i) 20-35% or preferably 25-30% by weight by weight on a dry weight basis of (S)-1-[(3-hydroxy-1-adamantyl)amino]acetyl-2-cyano-pyrrolidine in free form or in acid addition salt form, in free form or in acid addition salt form;
(ii) 25-70% by weight or preferably 35-50% by weight on a dry weight basis of a pharmaceutically acceptable microcrystalline cellulose such as Avicel PH 102;
(iii) 5-40% by weight or preferably 18-35% by weight on a dry weight basis of a pharmaceutically acceptable lactose;
(iv) 0-10% by weight or preferably 1-4% by weight on a dry weight basis of a pharmaceutically acceptable sodium starch glycolate; and
(v) 0.25- 6% by weight or preferably 0.5-4% by weight on a dry weight basis of a pharmaceutically acceptable magnesium stearate.

26. Process according to claim 23, wherein the blended composition used in step (a) is selected from the compositions of claims 9 to 19.

## Patentansprüche

1. Verpresste pharmazeutische Tablette oder direkt verpresste pharmazeutische Tablette, worin die Dispersion Teilchen enthält, die einen DPP-IV-Inhibitor umfassen, welcher (S)-1-[(3-Hydroxy-1-adamantyl)-amino]-acetyl-2-cyanopyrrolidin in freier Form oder in Form eines Säureadditionssalzes ist, und worin wenigstens 60 % der Teilchengrößenverteilung in der Tablette bei unter 250 µm liegen.

2. Verpresste pharmazeutische Tablette oder direkt verpresste pharmazeutische Tablette nach Anspruch 1, worin die Dispersion Teilchen enthält, die einen DPP-IV-Inhibitor umfassen, welcher (S)-1-[(3-Hydroxy-1-adamantyl)-amino]-acetyl-2-cyanopyrrolidin in freier Form oder in Form eines Säureadditionssalzes ist, und worin
i) wenigstens 60 % der Teilchengrößenverteilung in der Tablette bei unter 250 µm und vorzugsweise zwischen 10 und 250 µm liegen, und
ii) die Verhältnisse von Tablettendicke zu Tablettengewicht 0,002 bis 0,06 mm/mg oder 0,01 bis 0,03 mm/mg betragen.

3. Verpresste pharmazeutische Tablette oder direkt verpresste pharmazeutische Tablette nach Anspruch 1 oder 2, worin die Dispersion Teilchen enthält, die einen DPP-IV-Inhibitor umfassen, welcher (S)-1-[(3-Hydroxy-1-adamantyl)-amino]-acetyl-2-cyanopyrrolidin in freier Form oder in Form eines Säureadditionssalzes ist, und worin
i) wenigstens 60 % der Teilchengrößenverteilung in der Tablette bei unter 250 µm und vorzugsweise zwischen 10 und 250 µm liegen,
ii) der Wassergehalt der Tablette weniger als 10 % nach einer Woche bei 25 °C und 60 % Raumfeuchtigkeit beträgt, und
iii) die Verhältnisse von Tablettendicke zu Tablettengewicht 0,002 bis 0,06 mm/mg betragen.

4. Verpresste pharmazeutische Tablette oder direkt verpresste pharmazeutische Tablette nach einem der Ansprüche 1 bis 3, worin die Teilchengrößenverteilung in der Tablette zwischen 50 und 150 µm liegt.

5. Verpresste pharmazeutische Tablette oder direkt verpresste pharmazeutische Tablette nach einem der Ansprüche 1 bis 4, worin der Wassergehalt der Tablette weniger als 5 % nach einer Woche bei 25 °C und 60 % Raumfeuchtigkeit beträgt.

6. Verpresste pharmazeutische Tablette oder direkt verpresste pharmazeutische Tablette nach einem der Ansprüche 1 bis 5, worin die Verhältnisse von Tablettendicke zu Tablettengewicht 0,01 bis 0,03 mm/mg betragen.

7. Verpresste pharmazeutische Tablette oder direkt verpresste pharmazeutische Tablette nach einem der Ansprüche 1 bis 6, worin wenigstens 60 % oder wenigstens 80 % der Teilchengrößenverteilung in der Tablette zwischen 10 und 250 µm liegen.

8. Verpresste pharmazeutische Tablette oder direkt verpresste pharmazeutische Tablette nach einem der Ansprüche 1 bis 6, worin wenigstens 25 % oder wenigstens 35 % der Teilchengrößenverteilung in der Tablette zwischen 50 und 150 µm liegen.

9. Verpresste pharmazeutische Tablette oder direkt verpresste pharmazeutische Tablette nach einem der Ansprüche 1 bis 8, worin die Tablette umfasst
(a) 5 bis 60 Gew.-% auf einer Trockengewichtsbasis eines DPP-IV-Inhibitors, der (S)-1-[(3-Hydroxy-1-adamantyl)-amino]-acetyl-2-cyanopyrrolidin in freier Form oder in Form eines Säureadditionssalzes ist,
(b) 40 bis 95 Gew.-% auf einer Trockengewichtsbasis eines pharmazeutisch akzeptablen Verdünnungsmittels,
(c) 0 bis 20 Gew.-% auf einer Trockengewichtsbasis eines pharmazeutisch akzeptablen Zerfallhilfsmittels, und optional
(d) 0,1 bis 10 Gew.-% auf einer Trockengewichtsbasis eines pharmazeutisch akzeptablen Schmiermittels.

10. Verpresste pharmazeutische Tablette oder direkt verpresste pharmazeutische Tablette nach Anspruch 9, worin die Tablette umfasst
(a) 20 bis 40 Gew.-%, vorzugsweise 20 bis 35 Gew.-% auf einer Trockengewichtsbasis eines DPP-IV-Inhibitors, der (S)-1-[(3-Hydroxy-1-adamantyl)-amino]-acetyl-2-cyanopyrrolidin in freier Form oder in Form eines Säureadditionssalzes ist,
(b) 40 bis 95 Gew.-% auf einer Trockengewichtsbasis eines pharmazeutisch akzeptablen Verdünnungsmittels,
(c) 0 bis 10 Gew.-% auf einer Trockengewichtsbasis eines pharmazeutisch akzeptablen Zerfallhilfsmittels, und optional
(d) 0,25 bis 6 Gew.-% auf einer Trockengewichtsbasis eines pharmazeutisch akzeptablen Schmiermittels.

11. Verpresste pharmazeutische Tablette oder direkt verpresste pharmazeutische Tablette nach Anspruch 9 bis 10, worin die Tablette umfasst
(a) 20 bis 35 Gew.-% auf einer Trockengewichtsbasis eines (S)-1-[(3-Hydroxy-1-adamantyl)-amino]-acetyl-2-cyanopyrrolidins in freier Form oder in Form eines Säureadditionssalzes,
(b) 62 bis 78 Gew.-% auf einer Trockengewichtsbasis eines pharmazeutisch akzeptablen Verdünnungsmittels,
(c) 0 bis 10 Gew.-% auf einer Trockengewichtsbasis eines pharmazeutisch akzeptablen Zerfallhilfsmittels, und optional
(d) 0,1 bis 10 Gew.-% auf einer Trockengewichtsbasis eines pharmazeutisch akzeptablen Schmiermittels.

12. Verpresste pharmazeutische Tablette oder direkt verpresste pharmazeutische Tablette nach Anspruch 9 bis 11, worin die Tablette umfasst
(a) 22 bis 28 Gew.-% auf einer Trockengewichtsbasis eines (S)-1-[(3-Hydroxy-1-adamantyl)-amino]-acetyl-2-cyanopyrrolidins in freier Form oder in Form eines Säureadditionssalzes.

13. Verpresste pharmazeutische Tablette oder direkt verpresste pharmazeutische Tablette nach einem der Ansprüche 9 bis 10, umfassend
(a) 30 bis 35 Gew.-% auf einer Trockengewichtsbasis eines (S)-1-[(3-Hydroxy-1-adamantyl)-amino]-acetyl-2-cyanopyrrolidins in freier Form oder in Form eines Säureadditionssalzes, und
(b) 58 bis 72 Gew.-% auf einer Trockengewichtsbasis eines pharmazeutisch akzeptablen Verdünnungsmittels.

14. Verpresste pharmazeutische Tablette oder direkt verpresste pharmazeutische Tablette nach einem der Ansprüche 9 bis 13, umfassend
i) ein oder zwei Verdünnungsmittel, die ausgewählt sind aus mikrokristalliner Cellulose und Lactose
ii) die zwei Verdünnungsmittel mikrokristalline Cellulose und Lactose,
iii) 25 bis 70 Gew.-% und vorzugsweise 35 bis 55 Gew.-% auf einer Trockengewichtsbasis, einer pharmazeutisch akzeptablen mikrokristallinen Cellulose, oder
iv) 25 bis 70 Gew.-% und vorzugsweise 35 bis 55 Gew.-% auf einer Trockengewichtsbasis, einer pharmazeutisch akzeptablen mikrokristallinen Cellulose und 5 bis 40 Gew.-% und vorzugsweise 18 bis 35 Gew.-% auf einer Trockengewichtsbasis an Lactose.

15. Verpresste pharmazeutische Tablette oder direkt verpresste pharmazeutische Tablette nach einem der Ansprüche 9 bis 14, umfassend
(c) 1 bis 6 Gew.-% auf einer Trockengewichtsbasis eines pharmazeutisch akzeptablen Zerfallhilfsmittels, und/oder
(d) 0,1 bis 10 Gew.-% auf einer Trockengewichtsbasis eines pharmazeutisch akzeptablen Schmiermittels.

16. Verpresste pharmazeutische Tablette oder direkt verpresste pharmazeutische Tablette nach einem der Ansprüche 9 bis 13, umfassend
(a) 20 bis 35 Gew.-% auf einer Trockengewichtsbasis eines (S)-1-[(3-Hydroxy-1-adamantyl)-amino]-acetyl-2-cyanopyrrolidins in freier Form oder in Form eines Säureadditionssalzes,
(b) 25 bis 70 Gew.-% auf einer Trockengewichtsbasis einer pharmazeutisch akzeptablen mikrokristallinen Cellulose,
(c) 5 bis 40 Gew.-% auf einer Trockengewichtsbasis einer pharmazeutisch akzeptablen Lactose,
(d) 0 bis 10 Gew.-% auf einer Trockengewichtsbasis eines pharmazeutisch akzeptablen Natriumstärkeglycolats, und
(e) 0,25 bis 6 Gew.-% auf einer Trockengewichtsbasis an Magnesiumstearat.

17. Verpresste pharmazeutische Tablette oder direkt verpresste pharmazeutische Tablette nach einem der Ansprüche 9 bis 13, umfassend
(a) 30 bis 35 Gew.-% und vorzugsweise 30 bis 32 Gew.-% auf einer Trockengewichtsbasis eines (S)-1-[(3-Hydroxy-1-adamantyl)-amino]-acetyl-2-cyanopyrrolidins in freier Form oder in Form eines Säureadditionssalzes,
(b) 35 bis 50 Gew.-% und vorzugsweise 40 bis 45 Gew.-% auf einer Trockengewichtsbasis einer pharmazeutisch akzeptablen mikrokristallinen Cellulose,
(c) 18 bis 35 Gew.-% und vorzugsweise 20 bis 25 Gew.-% auf einer Trockengewichtsbasis einer pharmazeutisch akzeptablen Lactose,
(d) 1 bis 4 Gew.-% und vorzugsweise 1,5 bis 2,5 Gew.-% auf einer Trockengewichtsbasis eines pharmazeutisch akzeptablen Natriumstärkeglycolats, und
(e) 0,5 bis 4 Gew.-% und vorzugsweise 0,1 bis 2 Gew.-% auf einer Trockengewichtsbasis an Magnesiumstearat.

18. Verpresste pharmazeutische Tablette oder direkt verpresste pharmazeutische Tablette nach einem der Ansprüche 9 bis 13, umfassend
(a) 20 bis 35 Gew.-% und vorzugsweise 22 bis 28 Gew.-% auf einer Trockengewichtsbasis eines (S)-1-[(3-Hydroxy-1-adamantyl)-amino]-acetyl-2-cyanopyrrolidins in freier Form oder in Form eines Säureadditionssalzes,
(b) 35 bis 55 Gew.-% auf einer Trockengewichtsbasis einer pharmazeutisch akzeptablen mikrokristallinen Cellulose,
(c) 18 bis 35 Gew.-% auf einer Trockengewichtsbasis einer pharmazeutisch akzeptablen Lactose,
(d) 1 bis 4 Gew.-% auf einer Trockengewichtsbasis eines pharmazeutisch akzeptablen Natriumstärkeglycolats, und
(e) 0,5 bis 4 Gew.-% auf einer Trockengewichtsbasis an Magnesiumstearat.

19. Verpresste pharmazeutische Tablette oder direkt verpresste pharmazeutische Tablette nach einem der Ansprüche 9 bis 13, umfassend
(a) etwa 22 bis etwa 28 Gew.-% auf einer Trockengewichtsbasis eines (S)-1-[(3-Hydroxy-1-adamantyl)-amino]-acetyl-2-cyanopyrrolidins in freier Form oder in Form eines Säureadditionssalzes,
(b) etwa 45 bis etwa 50 Gew.-% auf einer Trockengewichtsbasis einer pharmazeutisch akzeptablen mikrokristallinen Cellulose,
(c) etwa 20 bis etwa 25 Gew.-% auf einer Trockengewichtsbasis einer pharmazeutisch akzeptablen Lactose,
(d) etwa 1,5 bis etwa 2,5 Gew.-% auf einer Trockengewichtsbasis eines pharmazeutisch akzeptablen Natriumstärkeglycolats, und
(e) etwa 0,1 bis etwa 2 Gew.-% auf einer Trockengewichtsbasis an Magnesiumstearat.

20. Verpresste pharmazeutische Tablette oder direkt verpresste pharmazeutische Tablette nach einem der Ansprüche 1 bis 19, worin
i) zwischen 0 und 10 min 85 bis 99,5 % des Wirkstoffs freigesetzt werden, und
ii) zwischen 10 und 15 min 90 bis 99,5 % des Wirkstoffs freigesetzt werden.

21. Verpresste pharmazeutische Tablette oder direkt verpresste pharmazeutische Tablette nach einem der Ansprüche 1 bis 19, worin die Teilchengrößenverteilung der pharmazeutischen Hilfsstoffe in der Tablette zwischen 5 und 400 µm liegt.

22. Verpresste pharmazeutische Tablette nach einem der Ansprüche 1 bis 21, die eine direkt verpresste Tablette ist.

23. Verfahren zur Herstellung einer direkt verpressten Tablette nach einem der Ansprüche 1 bis 22 in Einheitsdosierungsform, durch
(a) Vermischung als eine gewichtsprozentuale Menge auf einer Trockengewichtsbasis, von
(i) 6 bis 60 Gew.-% auf einer Trockengewichtsbasis eines DPP-IV-Inhibitors, und
(ii) wenigstens einem Hilfsstoff, ausgewählt aus einem Verdünnungsmittel, einem Zerfallhilfsmittel und einem Schmiermittel,
unter Bildung einer DPP-IV-Inhibitor-Formulierung in Form eines Tablettierpulvers, das direkt zu einer Tablette verpresst werden kann, und
(b) Verpressung der während der Stufe (a) hergestellten Formulierung unter Bildung der verpressten DPP-IV-Inhibitor-Tablette in Einheitsdosierungsform,
wobei dieser DPP-IV-Inhibitor (S)-1-[(3-Hydroxy-1-adamantyl)-amino]-acetyl-2-cyanopyrrolidin in freier Form oder in Form eines Säureadditionssalzes ist.

24. Verfahren zur Herstellung einer direkt verpressten Tablette nach einem der Ansprüche 1 bis 22 in Einheitsdosierungsform, durch
(a) Vermischung als eine gewichtsprozentuale Menge auf einer Trockengewichtsbasis, von
(i) 25 bis 35 Gew.-% auf einer Trockengewichtsbasis eines DPP-IV-Inhibitors,
(ii) 40 bis 95 Gew.-% auf einer Trockengewichtsbasis eines pharmazeutisch akzeptablen Verdünnungsmittels,
(iii) 0 bis 10 Gew.-% auf einer Trockengewichtsbasis eines pharmazeutisch akzeptablen Zerfallhilfsmittels, und
(iv) 0,25 bis 6 Gew.-% auf einer Trockengewichtsbasis eines pharmazeutisch akzeptablen Schmiermittels,
unter Bildung einer DPP-IV-Inhibitor-Formulierung in Form eines Tablettierpulvers, das direkt zu einer Tablette verpresst werden kann, und
(b) Verpressung der während der Stufe (a) hergestellten Formulierung unter Bildung der verpressten DPP-IV-Inhibitor-Tablette in Einheitsdosierungsform,
wobei dieser DPP-IV-Inhibitor (S)-1-[(3-Hydroxy-1-adamantyl)-amino]-acetyl-2-cyanopyrrolidin in freier Form oder in Form eines Säureadditionssalzes ist.

25. Verfahren nach Anspruch 24, worin die vermischte Formulierung umfasst:
(i) 20 bis 35 Gew.-% oder vorzugsweise 25 bis 30 Gew.-% auf einer Trockengewichtsbasis von (S)-1-[(3-Hydroxy-1-adamantyl)-amino]-acetyl-2-cyanopyrrolidin in freier Form oder in Form eines Säureadditionssalzes,
(ii) 25 bis 70 Gew.-% oder vorzugsweise 35 bis 50 Gew.-% auf einer Trockengewichtsbasis einer pharmazeutisch akzeptablen mikrokristallinen Cellulose, wie Avicel PH 102,
(iii) 5 bis 40 Gew.-% oder vorzugsweise 18 bis 35 Gew.-% auf einer Trockengewichtsbasis einer pharmazeutisch akzeptablen Lactose,
(iv) 0 bis 10 Gew.-% oder vorzugsweise 1 bis 4 Gew.-% auf einer Trockengewichtsbasis eines pharmazeutisch akzeptablen Natriumstärkeglycolats, und
(v) 0,25 bis 6 Gew.-% oder vorzugsweise 0,5 bis 4 Gew.-% auf einer Trockengewichtsbasis eines pharmazeutisch akzeptablen Magnesiumstearats.

26. Verfahren nach Anspruch 23, worin die in Stufe (a) verwendete vermischte Zusammensetzung aus den Zusammensetzungen nach den Ansprüchen 9 bis 19 ausgewählt ist.

## Revendications

1. Comprimé pharmaceutique formé par compression ou comprimé pharmaceutique formé par compression directe, dans lequel la dispersion contient des particules comprenant un inhibiteur de la DPP-IV qui est la (S)-1-[(3-hydroxy-1-adamantyl)amino]acétyl-2-cyanopyrrolidine sous forme libre ou sous forme de sel d'addition d'acide, et dans lequel au moins 60 % de la répartition granulométrique dans le comprimé est inférieure à 250 µm.

2. Comprimé pharmaceutique formé par compression ou comprimé pharmaceutique formé par compression directe selon la revendication 1, dans lequel la dispersion contient des particules comprenant un inhibiteur de la DPP-IV qui est la (S)-1-[(3-hydroxy-1-adamantyl)aminolacétyl-2-cyanopyrrolidine sous forme libre ou sous forme de sel d'addition d'acide, et dans lequel :
i) au moins 60 % de la répartition granulométrique dans le comprimé est inférieure à 250 µm, de préférence comprise entre 10 et 250 µm, et
ii) le rapport de l'épaisseur du comprimé au poids du comprimé est de 0,002 à 0,06 mm/mg ou de 0,01 à 0,03 mm/mg.

3. Comprimé pharmaceutique formé par compression ou comprimé pharmaceutique formé par compression directe selon la revendication 1 ou 2, dans lequel la dispersion contient des particules comprenant un inhibiteur de la DPP-IV qui est la (S)-1-[(3-hydroxy-1-adamantyl)amino]acétyl-2-cyanopyrrolidine sous forme libre ou sous forme de sel d'addition d'acide, et dans lequel :
i) au moins 60 % de la répartition granulométrique dans le comprimé est inférieure à 250 µm, de préférence comprise entre 10 et 250 µm,
ii) la teneur en eau du comprimé est inférieure à 10 % au bout d'une semaine passée à une température de 25°C et à une humidité ambiante de 60 %, et
iii) le rapport de l'épaisseur du comprimé au poids du comprimé est de 0,002 à 0,06 mm/mg.

4. Comprimé pharmaceutique formé par compression ou comprimé pharmaceutique formé par compression directe selon l'une quelconque des revendications 1 à 3, dans lequel la répartition granulométrique dans le comprimé est comprise entre 50 et 150 µm.

5. Comprimé pharmaceutique formé par compression ou comprimé pharmaceutique formé par compression directe selon l'une quelconque des revendications 1 à 4, dans lequel la teneur en eau du comprimé est inférieure à 5 % au bout d'une semaine passée à une température de 25°C et à une humidité ambiante de 60 %.

6. Comprimé pharmaceutique formé par compression ou comprimé pharmaceutique formé par compression directe selon l'une quelconque des revendications 1 à 5, dans lequel le rapport de l'épaisseur du comprimé au poids du comprimé est de 0,01 à 0,03 mm/mg.

7. Comprimé pharmaceutique formé par compression ou comprimé pharmaceutique formé par compression directe selon l'une quelconque des revendications 1 à 6, dans lequel au moins 60 % ou au moins 80 % de la répartition granulométrique dans le comprimé est comprise entre 10 et 250 µm.

8. Comprimé pharmaceutique formé par compression ou comprimé pharmaceutique formé par compression directe selon l'une quelconque des revendications 1 à 6, dans lequel au moins 25 % ou au moins 35 % de la répartition granulométrique dans le comprimé est comprise entre 50 et 150 µm.

9. Comprimé pharmaceutique formé par compression ou comprimé pharmaceutique formé par compression directe selon l'une quelconque des revendications 1 à 8, lequel comprend :
(a) 5-60 % en poids, sur une base en poids sec, d'un inhibiteur de la DPP-IV qui est la (S)-1-[(3-hydroxy-1-adamantyl)amino]acétyl-2-cyanopyrrolidine sous forme libre ou sous forme de sel d'addition d'acide ;
(b) 40-95 % en poids, sur une base en poids sec, d'un diluant pharmaceutiquement acceptable ;
(c) 0-20 % en poids, sur une base en poids sec, d'un délitant pharmaceutiquement acceptable ; et
(d) éventuellement, 0,1-10 % en poids, sur une base en poids sec, d'un lubrifiant pharmaceutiquement acceptable.

10. Comprimé pharmaceutique formé par compression ou comprimé pharmaceutique formé par compression directe selon la revendication 9, lequel comprend :
(a) 20-40 % en poids, de préférence 20-35 % en poids, sur une base en poids sec, d'un inhibiteur la DPP-IV qui est de la (S)-1-[(3-hydroxy-1-adamantyl)amino]acétyl-2-cyanopyrrolidine sous forme libre ou sous forme de sel d'addition d'acide ;
(b) 40-95 % en poids, sur une base en poids sec, d'un diluant pharmaceutiquement acceptable ;
(c) 0-10 % en poids, sur une base en poids sec, d'un délitant pharmaceutiquement acceptable ; et
(d) éventuellement, 0,25-6 % en poids, sur une base en poids sec, d'un lubrifiant pharmaceutiquement acceptable.

11. Comprimé pharmaceutique formé par compression ou comprimé pharmaceutique formé par compression directe selon la revendication 9 ou 10, lequel comprend :
(a) 20-35 % en poids, sur une base en poids sec, de (S)-1-[(3-hydroxy-1-adamantyl)amino]acétyl-2-cyanopyrrolidine sous forme libre ou sous forme de sel d'addition d'acide ;
(b) 62-78 % en poids, sur une base en poids sec, d'un diluant pharmaceutiquement acceptable ;
(c) 0-10 % en poids, sur une base en poids sec, d'un délitant pharmaceutiquement acceptable ; et
(d) éventuellement, 0,1-10 % en poids, sur une base en poids sec, d'un lubrifiant pharmaceutiquement acceptable.

12. Comprimé pharmaceutique formé par compression ou comprimé pharmaceutique formé par compression directe selon l'une quelconque des revendications 9 à 11, lequel comprend :
(a) 22-28 % en poids, sur une base en poids sec, de (S)-1-[(3-hydroxy-1-adamantyl)amino]acétyl-2-cyanopyrrolidine sous forme libre ou sous forme de sel d'addition d'acide.

13. Comprimé pharmaceutique formé par compression ou comprimé pharmaceutique formé par compression directe selon la revendication 9 ou 10, comprenant :
(a) 30-35 % en poids, sur une base en poids sec, de (S)-1-[(3-hydroxy-1-adamantyl)amino]acétyl-2-cyanopyrrolidine sous forme libre ou sous forme de sel d'addition d'acide, et
(b) 58-72 % en poids, sur une base en poids sec, d'un diluant pharmaceutiquement acceptable.

14. Comprimé pharmaceutique formé par compression ou comprimé pharmaceutique formé par compression directe selon l'une quelconque des revendications 9 à 13, comprenant :
i) un ou deux diluants choisis parmi la cellulose microcristalline et le lactose,
ii) les deux diluants cellulose microcristalline et lactose,
iii) 25-70 %, de préférence 35-55 % en poids, sur une base en poids sec, d'une cellulose microcristalline pharmaceutiquement acceptable, ou
iv) 25-70 %, de préférence 35-55 % en poids, sur une base en poids sec, d'une cellulose microcristalline pharmaceutiquement acceptable, et 5-40 %, de préférence 18-35 % en poids, sur une base en poids sec, de lactose.

15. Comprimé pharmaceutique formé par compression ou comprimé pharmaceutique formé par compression directe selon l'une quelconque des revendications 9 à 14, comprenant :
(c) 1-6 % en poids, sur une base en poids sec, d'un délitant pharmaceutiquement acceptable, et/ou
(d) 0,1-10 % en poids, sur une base en poids sec, d'un lubrifiant pharmaceutiquement acceptable.

16. Comprimé pharmaceutique formé par compression ou comprimé pharmaceutique formé par compression directe selon l'une quelconque des revendications 9 à 13, comprenant :
(a) 20-35 % en poids, sur une base en poids sec, de (S)-1-[(3-hydroxy-1-adamantyl)amino]acétyl-2-cyanopyrrolidine sous forme libre ou sous forme de sel d'addition d'acide ;
(b) 25-70 % en poids, sur une base en poids sec, d'une cellulose microcristalline pharmaceutiquement acceptable ;
(c) 5-40 % en poids, sur une base en poids sec, d'un lactose pharmaceutiquement acceptable ;
(d) 0-10 % en poids, sur une base en poids sec, d'un glycolate d'amidon sodique pharmaceutiquement acceptable ;
(e) 0,25-6 % en poids, sur une base en poids sec, de stéarate de magnésium.

17. Comprimé pharmaceutique formé par compression ou comprimé pharmaceutique formé par compression directe selon l'une quelconque des revendications 9 à 13, comprenant :
(a) 30-35 %, de préférence 30-32 % en poids, sur une base en poids sec, de (S)-1-[(3-hydroxy-1-adamantyl)amino]acétyl-2-cyanopyrrolidine sous forme libre ou sous forme de sel d'addition d'acide ;
(b) 35-50 %, de préférence 40-45 % en poids, sur une base en poids sec, d'une cellulose microcristalline pharmaceutiquement acceptable ;
(c) 18-35 %, de préférence 20-25 % en poids, sur une base en poids sec, d'un lactose pharmaceutiquement acceptable ;
(d) 1-4 %, de préférence 1,5-2,5 % en poids, sur une base en poids sec, d'un glycolate d'amidon sodique pharmaceutiquement acceptable ; et
(e) 0,5-4 %, de préférence 0,1-2 % en poids, sur une base en poids sec, de stéarate de magnésium.

18. Comprimé pharmaceutique formé par compression ou comprimé pharmaceutique formé par compression directe selon l'une quelconque des revendications 9 à 13, comprenant :
(a) 20-35 %, de préférence 22-28 % en poids, sur une base en poids sec, de (S)-1-[(3-hydroxy-1-adamantyl)amino]acétyl-2-cyanopyrrolidine sous forme libre ou sous forme de sel d'addition d'acide ;
(b) 35-55 % en poids, sur une base en poids sec, d'une cellulose microcristalline pharmaceutiquement acceptable ;
(c) 18-35 % en poids, sur une base en poids sec, d'un lactose pharmaceutiquement acceptable ;
(d) 1-4 % en poids, sur une base en poids sec, d'un glycolate d'amidon sodique pharmaceutiquement acceptable ; et
(e) 0,5-4 % en poids, sur une base en poids sec, de stéarate de magnésium.

19. Comprimé pharmaceutique formé par compression ou comprimé pharmaceutique formé par compression directe selon l'une quelconque des revendications 9 à 13, comprenant :
(a) d'environ 22 % à environ 28 % en poids, sur une base en poids sec, de (S)-1-[(3-hydroxy-1-adamantyl)amino]acétyl-2-cyanopyrrolidine sous forme libre ou sous forme de sel d'addition d'acide ;
(b) d'environ 45 % à environ 50 % en poids, sur une base en poids sec, d'une cellulose microcristalline pharmaceutiquement acceptable ;
(c) d'environ 20 % à environ 25 % en poids, sur une base en poids sec, d'un lactose pharmaceutiquement acceptable ;
(d) d'environ 1,5 % à environ 2,5 % en poids, sur une base en poids sec, d'un glycolate d'amidon sodique pharmaceutiquement acceptable ; et
(e) d'environ 0,1% à environ 2 % en poids, sur une base en poids sec, de stéarate de magnésium.

20. Comprimé pharmaceutique formé par compression ou comprimé pharmaceutique formé par compression directe selon l'une quelconque des revendications 1 à 19, dans lequel :
i) entre 0 et 10 minutes, de 85 à 99,5 % du principe actif est libéré, et
ii) entre 10 et 15 minutes, de 90 à 99,5 % du principe actif est libéré.

21. Comprimé pharmaceutique formé par compression ou comprimé pharmaceutique formé par compression directe selon l'une quelconque des revendications 1 à 19, dans lequel la répartition granulométrique des excipients pharmaceutiques dans le comprimé est comprise entre 5 et 400 µm.

22. Comprimé pharmaceutique formé par compression selon l'une quelconque des revendications 1 à 21, qui est un comprimé formé par compression directe.

23. Procédé de préparation d'un comprimé formé par compression directe selon l'une quelconque des revendications 1 à 22, sous forme galénique unitaire, qui comprend les étapes consistant à :
a) mélanger, en % en poids sur une base en poids sec :
(i) 6-60 % en poids, sur une base en poids sec, d'inhibiteur de la DPP-IV ; et
(ii) au moins un excipient choisi parmi un diluant, un délitant et un lubrifiant,
afin de former une formulation à base d'inhibiteur de la DPP-IV sous la forme d'une poudre à comprimer, capable d'être directement comprimée sous la forme d'un comprimé ; et
(b) comprimer la formulation préparée lors de l'étape (a) afin de former le comprimé d'inhibiteur de la DPP-IV comprimé, sous forme galénique unitaire,
ledit inhibiteur de la DPP-IV étant la (S)-1-[(3-hydroxy-1-adamantyl)amino]acétyl-2-cyanopyrrolidine sous forme libre ou sous forme de sel d'addition d'acide.

24. Procédé de préparation d'un comprimé formé par compression directe selon l'une quelconque des revendications 1 à 22, sous forme galénique unitaire, qui comprend les étapes consistant à :
a) mélanger, en % en poids sur une base en poids sec :
(i) 25-35 % en poids, sur une base en poids sec, d'inhibiteur de la DPP-IV ;
(ii) 40-95 % en poids, sur une base en poids sec, d'un diluant pharmaceutiquement acceptable ;
(iii) 0-10 % en poids, sur une base en poids sec, d'un délitant pharmaceutiquement acceptable ; et
(iv) 0,25-6 % en poids, sur une base en poids sec, d'un lubrifiant pharmaceutiquement acceptable,
afin de former une formulation à base d'inhibiteur de la DPP-IV sous la forme d'une poudre à comprimer, capable d'être directement comprimée sous la forme d'un comprimé ; et
(b) comprimer la formulation préparée lors de l'étape (a) afin de former le comprimé d'inhibiteur de la DPP-IV comprimé, sous forme galénique unitaire,
ledit inhibiteur de la DPP-IV étant la (S)-1-[(3-hydroxy-1-adamantyl)amino]acétyl-2-cyanopyrrolidine sous forme libre ou sous forme de sel d'addition d'acide.

25. Procédé selon la revendication 24, dans lequel la formulation mélangée comprend :
(i) 20-35 % en poids ou, de préférence, 25-30 % en poids, sur une base en poids sec, de (S)-1-[(3-hydroxy-1-adamantyl)amino]acétyl-2-cyanopyrrolidine sous forme libre ou sous forme de sel d'addition d'acide ;
(ii) 25-70 % en poids ou, de préférence, 35-50 % en poids, sur une base en poids sec, d'une cellulose microcristalline pharmaceutiquement acceptable telle que l'Avicel PH 102 ;
(iii) 5-40 % en poids ou, de préférence, 18-35 % en poids, sur une base en poids sec, d'un lactose pharmaceutiquement acceptable ;
(iv) 0-10 % en poids ou, de préférence, 1-4 % en poids, sur une base en poids sec, d'un glycolate d'amidon sodique pharmaceutiquement acceptable ; et
(v) 0,25-6 % en poids ou, de préférence, 0,5-4 % en poids, sur une base en poids sec, d'un stéarate de magnésium pharmaceutiquement acceptable.

26. Procédé selon la revendication 23, dans lequel la composition mélangée utilisée à l'étape (a) est choisie parmi les compositions des revendications 9 à 19.
